# EUROPÄISCHE PATENTSCHRIFT

(11) **EP 2 571 834 B1**
(45) Veröffentlichungstag und Bekanntmachung des Hinweises auf die Patenterteilung: **01.03.2017**
(21) Anmeldenummer: 11722300.8
(22) Anmeldetag: 21.04.2011
(51) Int. Cl.: C07C 7/04, C07C 11/06, B01D 3/00

(54) **VERFAHREN UND ANLAGE ZUR HERSTELLUNG OXYGENATARMER OLEFINSTRÖME**
METHOD AND SYSTEM FOR PRODUCING LOW-OXYGENATE OLEFIN FLOWS
PROCÉDÉ ET INSTALLATION DE PRÉPARATION DE COURANTS D'OLÉFINES PAUVRES EN OXYGÈNE

(30) Priorität: 20.05.2010 DE 102010022138
(43) Veröffentlichungstag der Anmeldung: 27.03.2013
(73) Patentinhaber: Air Liquide Global E&C Solutions Germany GmbH, 60439 Frankfurt am Main (DE)
(72) Erfinder: BACH, Hermann, 60439 Frankfurt (DE); RENNER, Thomas, 60388 Frankfurt (DE); ROTHÄMEL, Martin, 60388 Frankfurt (DE); WILKEN, Michael, 60439 Frankfurt (DE)
(86) Internationale Anmeldenummer: PCT/EP2011/002038
(87) Internationale Veröffentlichungsnummer: WO 2011/144288

(56) Entgegenhaltungen:
- EP-A1- 0 652 194
- EP-A1- 0 933 345
- US-A1- 2008 024 908
- US-A1- 2008 242 908
- US-B2- 7 678 958

## Beschreibung

### Gebiet der Erfindung

Die Erfindung betrifft ein Verfahren zur Herstellung eines Olefinstroms aus einem Kohlenwasserstoffgemisch-Einsatzstrom, wobei der Olefinstrom gegenüber dem Einsatzstrom hinsichtlich seines Gehalts an sauerstoffhaltigen organischen Verbindungen (Oxygenaten) abgereichert ist. Insbesondere betrifft die Erfindung ein Verfahren zur Herstellung und Bereitstellung eines Propylenstroms aus einem Kohlenwasserstoff-Einsatzstrom, der als Produktstrom bei der Olefinsynthese durch Umsetzung von Oxygenaten wie Alkoholen und/oder Ethern an Molekularsiebkatalysatoren erhalten wird. Die Erfindung betrifft auch eine Anlage zur Durchführung des erfindungsgemäßen Verfahrens.

### Stand der Technik

Kohlenwasserstoffverbindungen sind Grundstoffe der chemischen Industrie und Ausgangsstoffe für eine Vielzahl von Folgeprodukten. Typischerweise fallen die Kohlenwasserstoffverbindungen bei den primären Erzeugungsprozessen in Form von Gemischen an, die mittels Trennverfahren - vor allem der fraktionierten Destillation - in Einzelfraktionen oder Reinstoffe getrennt werden müssen. Die bei den herkömmlichen Verfahren verwendete Verschaltung der Trennapparate führt zu großen Abmessungen der einzelnen Ausrüstungsteile sowie einem hohen spezifischen Verbrauch an Betriebsmitteln.

Demnach kommt der optimalen Gestaltung des Trennprozesses eine große Bedeutung zu. Die Kohlenwasserstoff-Verbindungen sollen dabei in möglichst reiner Form ohne Anwesenheit von sauerstoffhaltigen organischen Verbindungen (Oxygenaten) erzeugt werden. Als Oxygenate werden Verbindungen bezeichnet, die ausschließlich aus Kohlenstoff, Wasserstoff und Sauerstoff zusammengesetzt sind; es handelt sich in der Regel um Alkohole oder Ether, die auch dem Benzin beigemischt werden können.

Insbesondere bei der Olefinsynthese durch Umsetzung von Oxygenaten wie Methanol und/oder Dimethylether (DME) an Molekularsiebkatalysatoren, wie sie beispielsweise in den europäischen Patentanmeldungen EP 0448000 A1 und EP 0882692 A1 und dem europäischen Patent EP 1289912 B1 beschrieben wird, ergibt sich die Aufgabe, geringe Mengen der als Edukt eingesetzten Oxygenate wie Methanol oder Dimethylether aus dem Reaktionsprodukt, einem an Olefinen reichen Kohlenwasserstoffstrom, in effizienter Weise zu entfernen, da die Oxygenate Katalysatorgifte bei der nachfolgenden Weiterverarbeitung der Olefine zu Polyolefinen darstellen. In Ullmann's Encyclopedia of Industrial Chemistry, Sixth Edition, 1998 Electronic Release, Stichwort "Polyolefins", Kapitel 2.3.1 "Propene", werden Grenzwerte für den Gehalt von Oxygenatkomponenten in einem Propylenstrom mit Polymerisationsreinheit (sogenanntes "polymer grade propylen") angegeben. So beträgt beispielsweise die tolerierbare Konzentration an Methanol in Propylen mit Polymerisationsreinheit maximal 5 Vol.-ppm.

Zur Abtrennung kleinerer Mengen bzw. geringerer Konzentrationen an Oxygenaten zur Erreichung von Polymerisationsreinheit eignen sich auch adsorptive Verfahren. Diese werden auch als nachgeschaltete Trennverfahren zum Beispiel nach einer destillativen Oxygenatabtrennung eingesetzt, um die erforderlichen Grenzwerte sicher einzuhalten.

Bei den in den oben genannten europäischen Patentanmeldungen bzw. Patenten gelehrten Verfahren wird die Olefinsynthese in Festbettreaktoren mit einem oder mehreren Katalysatorschüttungen durchgeführt. Mit fortschreitender Desaktivierung der eingesetzten Katalysatoren steigt die Oxygenatkonzentration im Olefinproduktstrom kontinuierlich an, so dass das einzusetzende Trennverfahren auch für zeitlich variierende Oxygenatkonzentrationen im zu behandelnden Einsatzstrom geeignet sein muss.

Nach dem Stand der Technik werden die Oxygenate durch eine klassisch verschaltete Destillation oder eine physikalische Wäsche von den Kohlenwasserstoff-Verbindungen getrennt. Dies ist aber insbesondere bei großtechnischen Anlagen sehr aufwändig und teuer. In den Schriften WO 03/020671, WO 03/020672 und WO 03/020678 werden Verfahren zur Extraktivdestillation von Olefinen beschrieben.

In der US-Patentschrift US 7678958 B2 wird ein Verfahren zur Entfernung von DME aus einem Olefinstrom mittels Destillation und nachgeschalteter Wasserwäsche beschrieben, der bei der Umsetzung von Oxygenaten zu Olefinen (OTO) erhalten wurde. Die Methode beinhaltet die Destillation des Olefinstroms, so dass DME gemeinsam mit dem Propan als Sumpfprodukt erhalten und somit aus dem Olefinstrom entfernt wird. Der Olefinstrom kann dann einer oder mehreren weiteren Destillationsstufen zugeführt werden, um letztlich einen Ethylenstrom und einen Propylenstrom mit Polymerisationsreinheit zu erhalten, wobei die DME-Konzentration in beiden Strömen jeweils 10 Gew.-ppm nicht übersteigt. Der DME wird durch Wasserwäsche aus dem Propanstrom entfernt, und der abgetrennte DME wird zu dem OTO-Reaktor zurückgeführt.

Die deutsche Patentschrift DE 102004052658 B3 lehrt ein Verfahren zur Entfernung von Oxygenaten aus Kohlenwasserstoffgemischen, bei dem der verbleibende Anteil der Oxygenate im Olefinstrom auf unter 1 ppm reduziert wird, sowie eine Trennung in Teilfraktionen erreicht wird, wobei die Apparateabmessungen und die spezifischen Betriebsmittelverbräuche minimiert werden. Die Lösung dieser Aufgabe erfolgt dadurch, dass das Gemisch aus Kohlenwasserstoffen und Oxygenaten in einem zweistufigen Trennprozess verarbeitet wird. Dabei liegt das Einsatz-Kohlenwasserstoffgemisch in einer zweiphasigen Form vor, wobei sich die schwereren Kohlenwasserstoffe in der flüssigen Phase befinden. Die beiden Phasen werden nicht wie herkömmlich gemeinsam einer Destillationskolonne aufgegeben, sondern jeweils separat in zwei Destillationsko-lonnen geführt. Aus der Flüssigphase wird in der ersten Kolonne eine leichte Fraktion abgetrennt, in der sich das Olefinprodukt und Oxygenate befinden. Die Gasphase wird zusammen mit der leichten Fraktion der ersten Kolonne der zweiten Kolonne aufgegeben. Bei dieser zweiten Kolonne handelt es sich um eine Extraktivdestillationskolonne.

Das Gemisch wird in einen leichten und einen schweren Kohlenwasserstoffschnitt getrennt. Dabei wird ein Lösungsmittel in den oberen Teil der Kolonne zugeführt, welches die Oxygenate löst. Damit sinkt der Gehalt an Oxygenaten im Vergleich zum Stand der Technik deutlich. Als Lösungsmittel werden verschiedene Komponenten gelehrt, so auch der als Edukt im vorgelagerten Methanol-zu-Propylen-(MTP^{®}-)Verfahren eingesetzte Alkohol Methanol. Vorteilhaft ist dabei, dass somit ein verfahrenseigener Stoff als Lösungsmittel eingesetzt wird, der ohnehin zur Verfügung steht, und das abgetrennte Oxygenat gemeinsam mit einem Restgehalt an Methanol zu der Olefinsynthese zurückgeführt werden kann.

Den im Stand der Technik gelehrten, destillativen Verfahren zur Oxygenatabtrennung ist gemein, dass das abzutrennende Oxygenat mit einer Kohlenwasserstofffraktion ähnlichen Siedepunkts anfällt, die nachfolgend durch einen weiteren Schritt, beispielsweise durch einen zusätzlichen Destillationsschritt, einen Extraktivdestillationsschritt, durch Waschen oder durch Adsorption oder durch eine Kombination mehrerer dieser Maßnahmen von der Kohlenwasserstofffraktion abgetrennt werden muss.

Die US-Patentanmeldung US 2008/242908 A1 lehrt ein Verfahren zur kontinuierlichen, destillativen Auftrennung eines olefinhaltigen Produktstroms, wobei der Einsatzstrom der Destillation aus einem Prozess stammt, bei dem Oxygenate mittels des MTO-Prozesses zu Olefinen umgewandelt werden. Dabei umfasst der Einsatzstrom Propen (Propylen), Propan und DME sowie weitere Komponenten, beispielsweise C₄₊-Komponenten wie Butane und Butene. Es ergibt sich für den Fachmann, dass der offenbarte Separator (20) nur eine Kombination aus mindestens zwei Destillationskolonnen sein kann. Folglich ist zur Abtrennung der C₄₊-Kohlenwasserstoffe eine separate, der C₃/DME-Trennung vorgeschaltete Trennkolonne erforderlich, die im "Separator" (20) durch einen C₄₊-Austrittsstrom angedeutet ist. Daraus folgt ferner, dass der DME-haltige C₃-Strom als Sumpfproduktstrom einer der C₄-Abtrennung nachgeschalteten, zweiten Trennkolonne erhalten wird. Die destillative Auftrennung des Systems Propylen-Propan-DME ist der eigentliche Gegenstand der Lehre der US 2008/242908 A1; sie schweigt hingegen hinsichtlich des Einflusses von C₄-Kohlenwasserstoffen auf die destillative Auftrennung des Systems Propylen-Propan-DME in einem nur eine Destillationskolonne umfassenden Trennsystem.

### Beschreibung der Erfindung

Der vorliegenden Erfindung liegt die Aufgabe zugrunde, ein alternatives Verfahren für die Herstellung eines Olefinstroms aus einem Kohlenwasserstoffgemisch-Einsatzstrom anzugeben, wobei der Olefinstrom gegenüber dem Einsatzstrom hinsichtlich seines Gehalts an Oxygenaten abgereichert ist. Das erfindungsgemäße Verfahren soll sich dabei durch apparative Einfachheit, hohe Energieeffizienz und das Fehlen von insbesondere prozessfremden Hilfsstoffen auszeichnen. Insbesondere ist es das Ziel der Erfindung, ein Verfahren zur Verfügung zu stellen, bei dem die Oxygenate in einem Stoffstrom anfallen, der entweder direkt weiterverwendet werden kann, oder dessen weitere, beispielsweise destillative oder adsorptive, Aufarbeitung weniger Aufwand erfordert als bei den im Stand der Technik bekannten Verfahren.

Die Lösung der erfindungsgemäßen Aufgabe ergibt sich im Wesentlichen aus den kennzeichnenden Merkmalen des Anspruchs 1 in Zusammenwirken mit den Merkmalen des Oberbegriffs dadurch, dass bei einem Verfahren zur Herstellung eines oxygenatarmen Olefinstroms aus einem Kohlenwasserstoffgemisch-Einsatzstrom, umfassend Propylen, Propan, die isomeren Butene und Butane, Dimethylether, sowie höhere (C₅₊-) paraffinische und olefinische Kohlenwasserstoffe in Spuren, die Destillation oder Rektifikation eingesetzt wird, wobei der Kohlenwasserstoffgemisch-Einsatzstrom einer Destillations- oder Rektifikationskolonne zugeführt wird, wobei im unteren Bereich der Kolonne ein an den Paraffinen gleicher und/oder höherer Kohlenstoffzahl und an den Olefinen höherer Kohlenstoffzahl angereicherter Sumpfproduktstrom abgezogen wird, wobei im oberen Bereich der Kolonne ein an Propylen angereicherter und an Dimethylether abgereicherter, propylenhaltiger Strom als Kopfproduktstrom abgezogen wird und wobei im Seitenabzug ein an Dimethylether angereicherter Strom abgezogen und aus dem Verfahren entfernt wird.

Weitere vorteilhafte Ausgestaltungen der Erfindung ergeben sich aus den Unteransprüchen.

Bei den im Stand der Technik bekannten, destillativen Verfahren zur Abtrennung von Oxygenaten aus Kohlenwasserstoffgemischen, die Olefine und Paraffine mit ähnlichem Siedepunkt und ähnlicher Molmasse wie das Oxygenat enthalten, bestand bislang in der Fachwelt das Vorurteil, dass das Oxygenat immer mit dem Sumpfprodukt die Destillation verlässt. Dies ist aufgrund der nachfolgend tabellarisch beispielhaft wiedergegebenen Zusammenstellung der Normalsiedepunkte und Molmassen für die C₃- und C₄-Systeme nachvollziehbar. Der Abstand der Normalsiedepunkte zwischen Propan und DME beträgt 18 °C, es ist der größte Siedepunktsabstand der in der Tabelle aufgeführten Verbindungen und bietet sich daher als Ansatzpunkt für die destillative Auftrennung des Kohlenwasserstoffgemisches an, das dann nach den im Stand der Technik beschriebenen Verfahren in ein leichtsiedendes Kopfprodukt, umfassend Propylen und Propan, und ein schwersiedendes Sumpfprodukt, umfassend DME und die isomeren Butane und Butene, aufgetrennt wird.

| **Komponente** | **Molmasse in g/mol** | **Normalsiedepunkt in °C¹⁾** |
|---|---|---|
| Propylen | 42,08 | - 47 |
| Propan | 44,10 | -42 |
| Dimethylether (DME) | 46,07 | -24 |
| i-Butan | 58,12 | -11 |
| 1-Buten | 56,11 | -6 |
| n-Butan | 58,12 | 0 |
| 2-Buten (E) | 56,11 | +1 |
| 2-Buten (Z) | 56,11 | +4 |

| | | |
|---|---|---|
| ¹⁾ Alle Daten aus NIST Chemistry WebBook, http://webbook.nist.gov/chemistry/, umgerechnet in °C. Der Normalsiedepunkt ist der Siedepunkt bei 101,25 kPa. | | |

Überraschenderweise wurde nun im Rahmen der vorliegenden Erfindung gefunden, dass die Möglichkeit besteht, bei einer nach einem thermischen Trennverfahren betriebenen Trennkolonne einen sowohl gegenüber dem Kopfprodukt als auch gegenüber dem Sumpfprodukt an Dimethylether angereicherten Stoffstrom über einen Seitenabzug zu gewinnen. Auf diese Weise lässt sich Dimethylether in effizienter Weise aus dem Trennverfahren entfernen, so dass ein an Dimethylether abgereicherter, propylenhaltiger Strom als Kopfproduktstrom erhalten werden kann, der hinsichtlich seiner Propylenkonzentration entweder bereits den Erfordernissen für die nachfolgende Weiterverwendung oder Weiterverarbeitung genügt, oder dessen weitere Reinigung weniger aufwändig durchgeführt werden kann als bei dem im Stand der Technik bekannten Verfahren. Im unteren Bereich der Trennkolonne wird ein an den Paraffinen gleicher und/oder höherer Kohlenstoffzahl und an den Olefinen höherer Kohlenstoffzahl angereicherter Sumpfproduktstrom abgezogen, der zumeist noch signifikante Restgehalte an Dimethylether enthält. In Abhängigkeit von der beabsichtigten Weiterverwendung oder Weiterverarbeitung stören diese aber unter Umständen nicht weiter. Wenn der Sumpfproduktstrom oder Teile davon beispielsweise im Falle der OTO-Reaktion zu der Olefinsynthese zurückgeführt werden, kann der Restgehalt an Dimethylether beim erneuten Durchgang durch den Synthesereaktor zusätzlich zu Olefinen umgesetzt werden.

Häufig macht die Bildung von Konzentrationsmaxima von Komponenten über die Höhe der Trennkolonne - der Fachmann spricht hier von Konzentrationsbäuchen - den Trennkolonnenbetrieb anfällig für Instabilitäten, die der Fachmann üblicherweise durch Anbringung eines sogenannten Seitenabzugs an der Kolonne löst. Hierzu wird an der Höhe der Trennkolonne, an der die betreffende Komponente in maximaler Konzentration vorliegt, eine kontinuierlich oder absatzweise betriebene Seitenabzugsleitung installiert, mit der dann gezielt die störende Komponente aus der Trennkolonne entfernt wird. Je nach Konzentration und Menge kann der Konzentrationsbauch komplett entfernt oder zumindest stabilisiert werden, so dass ein störungsfreier Betrieb der Trennkolonne wieder möglich wird. In der Regel wird aber der Fachmann versuchen, durch geeignete Auslegung des Trennverfahrens und der Trennkolonne die Bildung von Konzentrationsbäuchen zu vermeiden. Im Falle der vorliegenden Erfindung ist die Ausbildung eines solchen Konzentrationsbauches für Dimethylether günstig, da letzterer an dieser Stelle in konzentrierter Form aus der Trennkolonne abgezogen werden kann. Weiterhin ergibt sich der weitere Vorteil, dass nicht nur Dimethylether durch einen Seitenabzug abgezogen und aus dem Verfahren entfernt werden kann, sondern dass damit auch die Konzentration des Dimethylethers im oberen Teil der Trennkolonne gegenüber einem Betrieb ohne Seitenabzug reduziert werden kann.

Weitere bevorzugte Ausgestaltungen der Erfindung Im unteren Bereich der Kolonne wird ein an den Paraffinen gleicher und/oder höherer Kohlenstoffzahl und an den Olefinen höherer Kohlenstoffzahl angereicherter Sumpfproduktstrom abgezogen.

Der Kohlenwasserstoffgemisch-Einsatzstrom enthält demzufolge Propylen, Propan, die isomeren Butene und Butane, Dimethylether, sowie höhere (C₅₊-) paraffinische und olefinische Kohlenwasserstoffe in Spuren.

Die Auftrennung dieses Kohlenwasserstoffgemisch-Einsatzstroms ist von Bedeutung im Rahmen der Aufarbeitung des C₃-Produktstroms aus einer Olefinsynthesereaktion. Dabei können auch leichtere Kohlenwasserstoffe (C₂_-Fraktion) mit in die Trennkolonne gelangen; die Durchführung des erfindungsgemäßen Verfahrens wird hierdurch jedoch nicht beeinträchtigt.

In einer weiteren, bevorzugten Ausgestaltung ist vorgesehen, dass der Seitenabzug zum Abziehen des an Dimethylether angereicherten Stoffstroms mindestens einen theoretischen und/oder realen Boden oberhalb der Abzugsstelle des Sumpfproduktes und mindestens einen theoretischen und/oder realen Boden unterhalb der Abzugsstelle des Kopfproduktes angeordnet ist. Auf diese Weise erfolgt eine weitgehende Abreicherung des Dimethylether im Kopfprodukt, und eine signifikante Reduktion der Dimethylether-Konzentration im Sumpfprodukt gegenüber derjenigen im Seitenabzugsstrom. Aufgrund der höheren Anforderungen des Kopfproduktstroms hinsichtlich der Entfernung von Oxygenaten empfiehlt es sich, den Seitenabzug im unteren Kolonnenbereich, also oberhalb der Abzugsstelle des Sumpfproduktes, aber unterhalb der Einlassstelle für den Kohlenwasserstoffgemisch-Einsatzstrom anzuordnen.

Bevorzugt wird der Kohlenwasserstoffgemisch-Einsatzstrom als Produktstrom einer Olefinsynthesereaktion erhalten, bei der, ausgehend von Alkoholen und/oder Ethern, Olefine gebildet werden. Wie im eingangs diskutierten Stand der Technik bereits ausgeführt, wurden bereits unterschiedliche Verfahrensvarianten der Umwandlung von Oxygenaten zu Olefinen (OTO) beschrieben und haben teilweise bereits technische Reife erlangt, wie beispielsweise das Lurgi MTP^{®}-Verfahren. Bei der Aufarbeitung des C₃-Produkts aus den OTO-Verfahren stellt sich die Aufgabe, Restgehalte des als Einsatzstoff der Olefinsynthese dienenden DME aus dem Propylenprodukt zu entfernen, um die geforderten Reinheitsgrade des Propylenprodukts, beispielsweise Polymerisationsreinheit, zu erreichen. Das erfindungsgemäße Verfahren hat sich hierfür als besonders geeignet erwiesen.

In weiterbildender Ausgestaltung der Erfindung ist vorgesehen, den im Seitenabzug der Trennkolonne erhaltenen, an Dimethylether angereicherte Stoffstrom in die Olefinsynthesereaktion zurückzuführen. Auf diese Weise wird im Zusammenwirken des erfindungsgemäßen Verfahrens mit der vorgeschalteten Olefinsynthese, beispielsweise nach einem OTO-Verfahren, ein besonders hoher Wirkungsgrad erreicht, da die im Seitenabzug abgezogenen Oxygenate dem OTO-Reaktor in aufkonzentrierter Form aufgegeben werden, und somit in besonders effizienter Weise zu zusätzlichem Olefin umgesetzt werden können. Ferner kann ein Teil des bei Anwendung des erfindungsgemäßen Verfahrens erhaltenen Sumpfproduktes, das noch signifikante Oxygenatanteile, Paraffine mit gleicher oder höherer Kohlenstoffzahl wie das Olefin und Olefine höherer Kohlenstoffzahl enthält, ebenfalls dem OTO-Reaktor aufgegeben werden, da Paraffine häufig dem Reaktoreinsatz für OTO-Verfahren als Inert- bzw. Verdünnungsgas beigegeben werden, um die Wärmetönung der bei der Olefinsynthese ablaufenden Reaktionen besser kontrollieren zu können. Dabei wird wiederum ein Teil des im Sumpfprodukt verbliebenen Dimethylethers sowie ein Teil der Olefine höherer Kohlenstoffzahl zu zusätzlichem Olefin, bevorzugt Ethylen und/oder Propylen, umgesetzt.

Gemäß einer besonders bevorzugten Ausgestaltungsform der Erfindung wird der Kopfproduktstrom der Trennkolonne mindestens einem weiteren Aufarbeitungsschritt, bevorzugt einer Destillation bzw. Rektifikation oder einer Adsorptionsstufe, zugeführt. Auf diese Weise wird sichergestellt, dass die hohen Anforderungen hinsichtlich der Polymerisationsreinheit sicher eingehalten werden. Ein Beispiel für einen weiteren Aufarbeitungsschritt des oxygenatarmen Kopfproduktstroms ist die destillative Entfernung von Ethan, Ethylen und leichteren Komponenten, um einen Strom zu erhalten, der Propan, Propylen und geringe Restmengen an Dimethylether enthält und einem sogenannten Chemical-Grade Propylen entspricht, welches in verschiedenen Folgeprozessen direkt ohne weitere Aufarbeitung eingesetzt werden kann.

### Ausführungs- und Zahlenbeispiele

Weiterbildungen, Vorteile und Anwendungsmöglichkeiten der Erfindung ergeben sich auch aus der nachfolgenden Beschreibung von Ausführungs- und Zahlenbeispielen. Dabei bilden alle beschriebenen Merkmale für sich oder in beliebiger Kombination die Erfindung, unabhängig von ihrer Zusammenfassung in den Ansprüchen oder deren Rückbeziehung.

Die nachfolgenden Ausführungsbeispiele beziehen sich auf die destillative Aufarbeitung eines Produktstroms aus dem MTP^{®}-Verfahren, bei dem Olefine, insbesondere Propylen aus Methanol als Einsatzstoff über das Zwischenprodukt DME erzeugt werden. Die bei der Durchführung des MTP^{®}-Verfahrens zu wählenden Bedingungen und Verfahrensparameter sind dem Fachmann an sich bekannt; sie sind zudem in den eingangs bereits diskutierten Druckschriften EP 0448000 A1, EP 0882692 A1 und EP 1289912 B1 dargelegt.

### Beispiel 1 (Erfindung)

Es wurden Versuche zur destillativen Auftrennung eines Kohlenwasserstoffgemisch-Einsatzstroms durchgeführt, der einer vorgeschalteten, nach dem MTP^{®}-Verfahren betriebenen Olefinsynthese entstammte. Der aufzutrennende Kohlenwasserstoffstrom enthielt dabei Ethylen, Propylen, Propan, Butane und Butene sowie höhere Kohlenwasserstoffe, aber auch signifikante Anteile an DME. Die für die destillative Auftrennung verwendete Trennkolonne hatte eine Höhe von 4 m und war mit strukturierten Packungen des Typs Sulzer Mellapak® ausgerüstet. Unter den gewählten Verfahrensbedingungen besaß die Trennkolonne eine Trennleistung von 20 theoretischen Böden, die Höhe eines theoretischen Bodens (HETP-Wert) betrug mithin rund 0,20 m. Die Trennkolonne wurde unter einem Druck von 1,83 MPa(a) betrieben. Die Temperatur im Sumpf der Trennkolonne betrug 100 bis 120°C, die Temperatur am Kolonnenkopf betrug rund 40 °C. Das Rücklaufverhältnis betrug 25. Die Zulaufleitung für den Kohlenwasserstoffgemisch-Einsatzstrom befand sich in 2 m Höhe, gerechnet vom tiefsten Punkt der Trennkolonne, also etwa auf Höhe des 10. theoretischen Bodens, wobei Boden 1 dem Kolonnenkopf und Boden 20 dem Kolonnensumpf entspricht. Die Seitenabzugsleitung wurde auf Höhe des 14. Bodens in 1,20 m Höhe angeordnet.

Da der Kohlenwasserstoffgemisch-Einsatzstrom dem MTP^{®}-Synthesereaktor entstammte, veränderte sich seine Zusammensetzung in Abhängigkeit von der Laufzeit des MTP^{®}-Verfahrens aufgrund der fortschreitenden Desaktivierung des dort eingesetzten Katalysators. Die oben angegebenen Bedingungen bei seiner destillativen Auftrennung unterlagen daher ebenfalls zeitlichen Änderungen. In der nachfolgenden Tabelle werden die Betriebsbedingungen der Trennkolonne und die Zusammensetzungen der beteiligten Stoffströme für eine Laufzeit von 815 h zusammengestellt. Die Konzentrationen der Hauptkomponenten der Stoffströme wurden dabei mittels Kapillar-Gaschromatographie (GC) mit Wärmeleitfähigkeitsdetektor unter Verwendung von Standard-GC-Methoden bestimmt. Die Bestimmung der Spurenkomponenten, insbesondere der DME-Konzentration im Kopfprodukt, erfolgte dagegen mittels Gaschromatographie-Massenspektrometrie-Kopplung (GC/MS), ebenfalls nach Standardmethoden. Die Positionen des Zulaufs und der Seitenabzugsleitung und der Kolonnendruck wurden während der Versuchsdauer nicht verändert. Probenahmen der Destillationsprodukte erfolgten an zwei Stellen in der Trennkolonne (Boden Nr. 6 und Boden Nr. 14 = Position der Seitenabzugsleitung) sowie von Kopf- und Sumpfprodukt. Die entnommenen Proben bestätigten die Existenz eines Konzentrationsbauches für DME in der Nähe des 14. Bodens der Trennkolonne.

| **Boden Nr.** | **Höhe m** | **Position -** | **T °C** | **DME Vol.-%** | **Ethylen Vol.-%** | **Propan Vol.-%** | **Propylen Vol.-%** | **Butane + Butene Vol.-%** |
|---|---|---|---|---|---|---|---|---|
| 10 | 2.0 | Mitte, **Zulauf** | 60 | 6.30 | 18.50 | 0.86 | 29.38 | 24.32 |
| | | | | | | | | |
| 1 | 4.0 | **Kopf** | 39 | 0.03 | 36.29 | 1.70 | 57.02 | <0.01 |
| 6 | 2.8 | oben | 41 | 2.67 | 15.18 | 3.86 | 76.74 | 0.02 |
| 14 | 1.2 | unten, **Seitenabzug** | 77 | 34.86 | 0.06 | 4.06 | 55.82 | 5.00 |
| 20 | 0 | Sumpf | 114 | 10.49 | 0.14 | <0.01 | 0.38 | 47.05 |

Deutlich ist zu erkennen, dass über den Seitenabzug ein an DME angereicherter Stoffstrom abgezogen wird, während das Kopfprodukt der Destillation fast vollständig aus Ethylen und Propylen besteht und nur noch DME-Spuren, sowie geringfügige Anteile an Propan und Spuren an Butanen und Butenen enthält. Auch das Sumpfprodukt enthält noch signifikante Anteile an DME; diese stören dort aber nicht, da das Sumpfprodukt, wie in der Beschreibung der Erfindung ausgeführt, mindestens teilweise zum MTP^{®}-Synthesereaktor zurückgeführt werden kann.

### Vergleichsbeispiel

Vor der Durchführung der oben beschriebenen, erfindungsgemäßen Destillationsversuche wurden Versuche in der derselben Trennkolonne durchgeführt, wobei die Betriebsbedingungen der Trennkolonne und auch die Zusammensetzung des Kohlenwasserstoffgemisch-Einsatzstroms weitgehend den oben beschriebenen Bedingungen entsprachen. Allerdings war die Trennkolonne dabei nicht mit einem Seitenabzug ausgerüstet. Es konnte zwar ein Großteil des DME mit dem Sumpfprodukt abgetrennt werden, im propylenhaltigen Kopfprodukt traten aber weiterhin Konzentrationen von bis zu 0,2 Vol-% DME auf, die auch durch Modifikation der Betriebsbedingungen der Trennkolonne nicht weiter reduziert werden konnten.

### Beispiel 2 (Erfindung)

Es wurden Langzeitversuche zur kontinuierlichen Olefinsynthese nach dem MTP^{®}-Verfahren durchgeführt, wobei wiederum dem MTP^{®}-Synthesereaktor eine gemäß Beispiel 1 betriebene Trennkolonne nachgeschaltet war. Aufgrund der fortschreitenden Alterung bzw. Desaktivierung des im Synthesereaktor eingesetzten Katalysators wird DME nicht mehr vollständig umgesetzt, daher steigt die DME-Konzentration im Produktstrom des Synthesereaktors kontinuierlich an. Die Trennkolonne wurde dabei für unterschiedliche Versuchszeiträume erfindungsgemäß mit Seitenabzug bzw. zum Vergleich ohne Seitenabzug betrieben. In der nachfolgenden Tabelle werden die dabei gemessenen DME-Konzentrationen im Kopfprodukt in Abhängigkeit von der Betriebsdauer der Versuchsanlage und dem Betriebszustand des Seitenabzugs dargestellt. Zum Vergleich ist auch die DME-Konzentration im Kohlenwasserstoffgemisch-Einsatzstrom in Abhängigkeit von der Laufzeit aufgeführt, die der DME-Konzentration am Ausgang des Synthesereaktors entspricht.

| **Laufzeit** | **h** | **23** | **47** | **71** | **95** | **119** | **143** | **167** | **179** |
|---|---|---|---|---|---|---|---|---|---|
| **DME im Einsatzstr.** | **Vol.-%** | 0.11 | 0.14 | 0.16 | 0.19 | 0.22 | 0.30 | 0.35 | 0.45 |
| **DME im Kopfprodukt** | **Vol.-%** | 0.01 | 0.01 | 0.01 | 0.01 | 0.01 | 0.01 | 0.02 | 0.02 |
| **Seitenabzug aktiv ^{#)}** | +/- | - | - | - | - | - | - | - | - |
| | | | | | | | | | |
| **Laufzeit** | **h** | **191** | **203** | **215** | **227** | **239** | **263** | **275** | **299** |
| **DME im Einsatzstr.** | **Vol.-%** | 0.63 | 0.42 | 0.81 | 0.55 | 0.73 | 0.71 | 0.85 | 0.99 |
| **DME im Kopfprodukt** | **Vol.-%** | 0.02 | 0.02 | 0.02 | 0.02 | 0.02 | 0.05 | 0.09 | 0.12 |
| **Seitenabzug aktiv ^{#}** | +/- | - | - | - | - | - | - | - | - |
| | | | | | | | | | |
| **Laufzeit** | **h** | **311** | **323** | **335** | **347** | **359** | **371** | **383** | **395** |
| **DME im Einsatzstr.** | **Vol.-%** | 1.08 | 1.46 | 1.35 | 1.79 | 1.60 | 1.89 | 1.80 | 1.71 |
| **DME im Kopfprodukt** | **Vol.-%** | **0.06** | **0.07** | **0.07** | **0.09** | **0.06** | **0.08** | **0.09** | **0.10** |
| **Seitenabzug aktiv ^{#}** | +/- | + | + | + | + | + | + | + | + |
| | | | | | | | | | |
| **Laufzeit** | **h** | **407** | **419** | **431** | **443** | **455** | **467** | **479** | **491** |
| **DME im Einsatzstr.** | **Vol.-%** | 1.95 | 2.19 | 2.29 | 2.40 | 2.27 | 2.73 | 3.02 | 3.08 |
| **DME im Kopfprodukt** | **Vol.-%** | **0.07** | **0.05** | **0.05** | **0.05** | **0.04** | **0.07** | 0.24 | 0.28 |
| **Seitenabzug aktiv ^{#}** | +/- | + | + | + | + | + | + | - | - |
| | | | | | | | | | |
| **Laufzeit** | **h** | **503** | | | | | | | |
| **DME im Einsatzstr.** | **Vol.-%** | 3.66 | | | | | | | |
| **DME im Kopfprodukt** | **Vol.-%** | 0.33 | | | | | | | |
| **Seitenabzug aktiv ^{#}** | +/- | - | - | | | | | | |

| | | | | | | | | | |
|---|---|---|---|---|---|---|---|---|---|
| ^{#)} + aktiv, - nicht aktiv | | | | | | | | | |

Es ist erkennbar, dass bei Betrieb des Seitenabzugs bei Laufzeiten zwischen 311 und 467 h die Konzentration des DME im Kopfprodukt deutlich abgesenkt und sicher unter einem Wert von 0,1 Vol.-% gehalten werden kann, trotz der kontinuierlich ansteigenden DME-Konzentration am Ausgang des Synthesereaktors. Nach Außerbetriebnahme des Seitenabzugs bei Laufzeiten größer als 467 h steigt die DME-Konzentration im Kopfprodukt rapide an.

Da mit dem erfindungsgemäßen Verfahren die DME-Konzentration im Kopfprodukt der Trennkolonne sicher und konstant unter einem vorgegebenen Grenzwert gehalten werden kann, bietet es sich zur weiteren Absenkung der DME-Konzentration an, den Kopfproduktstrom einem weiteren Trennverfahren, beispielsweise der Adsorption, zuzuführen, das bei konstanter Konzentration der abzuscheidenden Komponente besonders gut arbeitet.

### Gewerbliche Anwendbarkeit

Mit der Erfindung wird ein Verfahren zur Herstellung eines Olefinstroms aus einem Kohlenwasserstoffgemisch-Einsatzstrom zur Verfügung gestellt, das sich im Vergleich zu den im Stand der Technik bekannten Verfahren durch seine apparative Einfachheit sowie durch die Abwesenheit zusätzlicher, insbesondere verfahrensfremder, Extraktionsmittel auszeichnet. Die Vorteile der im Stand der Technik bekannten Verfahren hinsichtlich des Einsatzes bewährter Destillations- bzw. Rektifikationsverfahren bleiben weiter bestehen. Im Falle der Kombination des erfindungsgemäßen Verfahrens mit einer vorgeschalteten Olefinsynthese, beispielsweise nach einem OTO-Verfahren, ergeben sich zusätzliche Vorteile durch die stoffliche Nutzung der im Seitenabzugsproduktstrom bzw. im Sumpfproduktstrom verbliebenen Oxygenate bei der Rückführung dieser Ströme zum Olefinsynthesereaktor.

## Patentansprüche

1. Verfahren zur Herstellung eines an Dimethylether verarmten propylenhaltigen Stroms aus einem Kohlenwasserstoffgemisch-Einsatzstrom, umfassend Propylen, Propan, die isomeren Butene und Butane, Dimethylether, sowie höhere (C₅₊-) paraffinische und olefinische Kohlenwasserstoffe in Spuren, mittels Destillation oder Rektifikation, wobei der Kohlenwasserstoffgemisch-Einsatzstrom einer Destillations- oder Rektifikationskolonne zugeführt wird, wobei im unteren Bereich der Kolonne ein an den Paraffinen gleicher und/oder höherer Kohlenstoffzahl und an den Olefinen höherer Kohlenstoffzahl angereicherter Sumpfproduktstrom abgezogen wird und wobei im oberen Bereich der Kolonne ein an Propylen angereicherter und an Dimethylether abgereicherter, propylenhaltiger Strom als Kopfproduktstrom abgezogen wird, **dadurch gekennzeichnet, dass** im Seitenabzug ein an Dimethylether angereicherter Strom abgezogen und aus dem Verfahren entfernt wird.

2. Verfahren nach Anspruch 1, **dadurch gekennzeichnet, dass** der Seitenabzug mindestens einen theoretischen und/oder realen Boden oberhalb der Abzugsstelle des Sumpfproduktes und mindestens einen theoretischen und/oder realen Boden unterhalb der Abzugsstelle des Kopfproduktes angeordnet ist.

3. Verfahren nach Anspruch 1, **dadurch gekennzeichnet, dass** der Kohlenwasserstoffgemisch-Einsatzstrom als Produktstrom einer Olefinsynthesereaktion erhalten wird, bei der, ausgehend von Alkoholen und/oder Ethern, Olefine gebildet werden.

4. Verfahren nach Anspruch 1, **dadurch gekennzeichnet, dass** der im Seitenabzug der Kolonne erhaltene, an Dimethylether angereicherte Strom in die Olefinsynthesereaktion zurückgeführt wird.

5. Verfahren nach Anspruch 1, **dadurch gekennzeichnet, dass** der Kopfproduktstrom mindestens einem weiteren Aufarbeitungsschritt, bevorzugt einer Destillation, Rektifikation oder Adsorption, zugeführt wird, durch den seine Dimethylether-Konzentration weiter verringert wird.

## Claims

1. A process for producing a propylene-containing stream depleted of dimethyl ether from a hydrocarbon mixture feed stream, comprising propylene, propane, the isomeric butenes and butanes, dimethyl ether as well as higher (C₅₊) paraffinic and olefinic hydrocarbons in traces, by means of distillation or rectification, wherein the hydrocarbon mixture feed stream is supplied to a distillation or rectification column, wherein in the lower region of the column a bottom product stream enriched in the paraffins of equal and/or higher carbon number and in the olefins of higher carbon number is withdrawn and wherein in the upper region of the column a propylene-containing stream enriched in propylene and depleted of dimethyl ether is withdrawn as top product stream, **characterized in that** in the side draw a stream enriched in dimethyl ether is withdrawn and removed from the process.

2. The process according to claim 1, **characterized in that** the side draw is arranged at least one theoretical and/or real tray above the withdrawal point of the bottom product and at least one theoretical and/or real tray below the withdrawal point of the top product.

3. The process according to claim 1, **characterized in that** the hydrocarbon mixture feed stream is obtained as product stream of an olefin synthesis reaction in which, proceeding from alcohols and/or ethers, olefins are formed.

4. The process according to claim 1, **characterized in that** the stream enriched in dimethyl ether, which is obtained in the side draw of the column, is recirculated into the olefin synthesis reaction.

5. The process according to claim 1, **characterized in that** the top product stream is supplied to at least one further processing step, preferably to a distillation, rectification or adsorption, by which its dimethyl ether concentration is reduced further.

## Revendications

1. Procédé de préparation d'un courant contenant du propylène appauvri en oxyde diméthylique à partir d'un courant de charge de mélange d'hydrocarbures, comprenant du propylène, du propane, les butènes isomères et du butane, de l'oxyde diméthylique, ainsi que des hydrocarbures paraffiniques supérieurs (C₅₊) et oléfiniques en traces, au moyen d'une distillation ou d'une rectification, le courant de charge de mélange d'hydrocarbures étant envoyé à une colonne de distillation ou de rectification, dans lequel on soutire, dans la partie inférieure de la colonne, un courant de produit de queue enrichi en les paraffines ayant le même nombre de carbones et/ou un nombre plus grand de carbones et en les oléfines ayant un nombre de carbones plus grand et on soutire, comme courant de produit de tête, dans la partie supérieure de la colonne, un courant contenant du propylène enrichi en propylène et appauvri en oxyde diméthylique, **caractérisé en ce qu'**on soutire, dans un soutirage latéral, un courant enrichi en oxyde diméthylique et on l'élimine du procédé.

2. Procédé suivant la revendication 1, **caractérisé en ce que** le soutirage latéral est au moins un plateau théorique et/ou un plateau réel au-dessus du point de soutirage du produit de queue et au moins un plateau théorique et/ou un plateau réel en dessous du point de soutirage du produit de tête.

3. Procédé suivant la revendication 1, **caractérisé en ce que** le courant de charge de mélange d'hydrocarbures est obtenu comme courant de produit d'une réaction de synthèse d'oléfines, dans laquelle on forme des oléfines en partant d'alcool et/ou d'étheroxyde.

4. Procédé suivant la revendication 1, **caractérisé en ce qu'**on retourne à la réaction de synthèse d'oléfines, le courant enrichi en oxyde diméthylique obtenu dans le soutirage latéral de la colonne.

5. Procédé suivant la revendication 1, **caractérisé en ce qu'**on envoie le courant de produit de tête au moins à un autre stade de traitement, de préférence à une distillation et à une rectification ou à une adsorption, par lequel sa concentration en oxyde diméthylique est encore diminuée.
